# EUROPEAN PATENT SPECIFICATION

(11) **EP 1 591 122 B1**
(45) Date of publication and mention of the grant of the patent: **26.09.2012**
(21) Application number: 05012711.7
(22) Date of filing: 18.06.2001
(51) Int. Cl.: A61K 31/663, A61P 19/08, A61P 19/10

(54) **Method of administering bisphosphonates**
Methode zur Verabreichung von Biphosphonaten
Méthode pour l'administration de biphosphonates

(30) Priority: 20.06.2000 US 597135; 09.02.2001 US 267689 P
(43) Date of publication of application: 02.11.2005
(62) Divisional of application: 01940580.2
(73) Proprietor: Novartis AG, 4056 Basel (CH); Novartis Pharma GmbH, 1230 Wien (AT)
(72) Inventor: Horowitz, Zebulun D., Basking Ridge, NJ 07920 (US); Richardson, Peter C., Far Hills, NJ 07931 (US); Trechsel, Ulrich, 7242 Luzein (CH)
(74) Representative: Warner, James Alexander

(56) References cited:
- WO-A-00/71104
- WO-A-99/15155
- WO-A-99/18972
- DE-A- 19 853 483
- US-A- 6 015 801

## Description

This invention relates to bisphosphonates, in particular to the pharmaceutical use of bisphosphonates in the treatment of conditions of abnormally increased bone turnover, such as osteoporosis.

Bisphosphonates are widely used to inhibit osteoclast activity in a variety of both benign and malignant diseases in which bone resorption is increased. Thus bisphosphonates have recently become available for long-term treatment of patients with Multiple Myeloma (MM). These pyrophosphate analogs not only reduce the occurrence of skeletal related events but they also provide patients with clinical benefit and improve survival. Bisphosphonates are able to prevent bone resorption *in vivo;* the therapeutic efficacy of bisphosphonates has been demonstrated in the treatment of Paget's disease of bone, tumour-induced hypercalcemia and, more recently, bone metastasis and multiple myeloma (MM) (for review see Fleisch H 1997 Bisphosphonates clinical. In Bisphosphonates in Bone Disease. From the Laboratory to the Patient. Eds: The Parthenon Publishing Group, New York/London pp 68-163). The mechanisms by which bisphosphonates inhibit bone resorption are still poorly understood and seem to vary according to the bisphosphonates studied. Bisphosphonates have been shown to bind strongly to the hydroxyapatite crystals of bone, to reduce bone turn-over and resorption, to decrease the levels of hydroxyproline or alkaline phosphatase in the blood, and in addition to inhibit both the activation and the activity of osteoclasts.

In addition bisphosphonates have been proposed for use in the treatment of osteoporosis. Thus for instance, as described in USP 4,812,304 (Procter & Gamble) a method has been proposed for treating or preventing osteoporosis in humans comprising administering to a subject afflicted with or at risk to osteoporosis a bone cell activating compound and a bone resorption inhibiting polyphosphonate according to a regime consisting of one or more cycles, whereby each cycle consists of: (a) a bone activating period of from about 1 day to about 5 days during which a bone cell activating amount of a bone cell activating compound is administered to said subject; followed by (b) a bone resorption inhibition period of from about 10 days to about 20 days during which ethane-1-hydroxy-1,1-diphosphonic acid, or a pharmaceutically acceptable salt or ester thereof, is administered daily to said subject in an amount from about 0.25 mgP/kg/day to about 3.3mgP/kg/day; followed by (c) a rest period of from about 70 days to about 180 days during which the subject receives neither a bone cell activating compound nor a bone resorption inhibiting polyphophonate.

Also, for example, USP 4,761,406 (Procter & Gamble) proposes a method for treating osteoporosis, in humans or lower animals afflicted with or at risk of osteoporosis, comprising administering to said human or lower animal an effective amount of a bone resorption inhibiting polyphosphonate according to the following schedule: (a) a period of from about 1 day to about 90 days during which said bone resorption inhibiting polyphosphonate is administered daily in a limited amount, followed by (b) a rest period of from about 50 days to about 120 days, and (c) repeating (a) and (b) two or more times where a net increase in bone mass of said human or animal results.

Surprisingly we have now found that bisphosphonates, in particular more potent nitrogen-containing bisphosphonates, can be used for prolonged inhibition of bone resorption in conditions of abnormally increased bone turnover by intermittent administration, wherein the periods between bisphosphonate administrations are longer than was previously considered appropriate to achieve satisfactory treatment. In particular and contrary to expectation we have found that satisfactory treatment results can be obtained even when the dosing intervals greatly exceed the natural bone remodelling cycle.

The invention further provides Zoledronic acid or a pharmaceutically acceptable salt thereof or any hydrate thereof for use in a method of treating osteoporosis in which the zoledronic acid or the pharmaceutically acceptable salt therefore or the hydrate thereof is administered intravenously and intermittently and in which the period between administrations is at least about 6 months.

Conditions of abnormally increased bone turnover which may be treated in accordance with the present invention include: treatment of postmenopausal osteoporosis, e.g. to reduce the risk of osteoporotic fractures; prevention of postmenopausal osteoporosis, e.g. prevention of postmenopausal bone loss; treatment or prevention of male osteoporosis; treatment or prevention of corticosteroid-induced osteoporosis and other forms of bone loss secondary to or due to medication, e.g. diphenylhydantoin, thyroid hormone replacement therapy; treatment or prevention of bone loss associated with immobilisation and space flight; treatment or prevention of bone loss associated with rheumatoid arthritis, osteogenesis imperfecta, hyperthyroidism, anorexia nervosa, organ transplantation, joint prosthesis loosening, and other medical conditions. For example, such other medical conditions may include treatment or prevention of periarticular bone erosions in rheumatoid arthritis; treatment of osteoarthritis, e.g. prevention/treatment of subchondral osteosclerosis, subchondral bone cysts, osteophyte formation, and of osteoarthritic pain, e.g. by reduction in intra-osseous pressure; treatment or prevention of hypercalcemia resulting from excessive bone resorption secondary to hyperparathyroidism, thyrotoxicosis, sarcoidosis or hypervitaminosis D.

Thus in the present description the terms "treatment" or "treat" refer to both prophylactic or preventative treatment as well as curative or disease modifying treatment, including treatment of patients at risk of contracting the disease or suspected to have contracted the disease as well as patients who are ill or have been diagnosed as suffering from a disease or medical condition. In particularly preferred embodiments the invention may be used for the prophylactic treatment of osteoporosis and similar diseases. Thus for example, zoledronic acid, or a pharmaceutically acceptable salt thereof, or any hydrate thereof may be administered to individuals at risk of developing osteoporosis on a regular basis at dosing intervals of at least about 6 months, e.g. bisphosphnates may be administered routinely to postmenopausal women at dosing intervals of once every 6 months or less frequently, e.g. annually.

In accordance with the present invention the zoledronic acid, or a pharmaceutically acceptable salt thereof, or any hydrate thereof dosing interval is at least about 6 months, e.g. once every 180 days, or less frequently, conveniently once a year, or at any interval in between, e.g. once every 7, 8, 9, 10, or 11 months. Dosing intervals of greater than once per year may be used, e.g. about once every 18 months or about once every 2 years, or even less frequently, e.g. a frequency of up to about once every 3 years or less often.

The most preferred N-bisphosphonate for use in the invention is 2-(imidazol-1yl)-1-hydroxyethane-1,1-diphosphonic acid (zoledronic acid) or a pharmacologically acceptable salt thereof.

Pharmacologically acceptable salts are preferably salts with bases, conveniently metal salts derived from groups Ia, Ib, IIa and IIb of the Periodic Table of the Elements, including alkali metal salts, e.g. potassium and especially sodium salts, or alkaline earth metal salts, preferably calcium or magnesium salts, and also ammonium salts with ammonia or organic amines.

Especially preferred pharmaceutically acceptable salts are those where one, two, three or four, in particular one or two, of the acidic hydrogens of the bisphosphonic acid are replaced by a pharmaceutically acceptable cation, in particular sodium, potassium or ammonium, in first instance sodium.

A very preferred group of pharmaceutically acceptable salts is characterized by having one acidic hydrogen and one pharmaceutically acceptable cation, especially sodium, in each of the phosphonic acid groups.

The zoledronic acid, or a pharmaceutically acceptable salt thereof, or any hydrate thereof derivatives specifically mentioned above are well known from the literature. This includes their manufacture -hydroxy-2-(imidazol-1-yl)-ethane-1,1-diphosphonic acid is prepared as described e.g. in US patent 4,939,130.

The zoledronic acid, or a pharmaceutically acceptable salt thereof, or any hydrate thereof (hereinafter referred to as the Agents of the Invention) may be used in the form of an isomer or of a mixture of isomers where appropriate, typically as optical isomers such as enantiomers or diastereoisomers or geometric isomers, typically cis-trans isomers. The optical isomers are obtained in the form of the pure antipodes and/or as racemates.

The Agents of the Invention can also be used in the form of their hydrates or include other solvents used for their crystallisation.

The Agents of the Invention are preferably used in the form of pharmaceutical compositions that contain a therapeutically effective amount of active ingredient optionally together with or in admixture with inorganic or organic, solid or liquid, pharmaceutically acceptable carriers which are suitable for administration.

The Agents of the Invention may be administered alone or in combination with other bone active drugs, either in fixed combinations or separately both physically and in time, including hormones, such as a steroid hormone, e.g. an estrogen; a partial estrogen agonist, or estrogengestagen combination;, a calcitonin or an analogue or derivative thereof, e.g. salmon, eel or human calcitonin parathyroid hormone or analogues thereof , e.g. e.g. PTH (1-84), PTH (1-34), PTH (1-36), PTH (1-38), PTH (1-31)NH₂ or PTS 893; a SERM (Selective Estrogen Receptor Modulator) e.g. raloxifene, lasofoxifene, TSE-424, FC1271, Tibolone (Livial ®); vitamin D or an analog. Such additional bone active drugs may be administered more frequently than the bisphosphonate.

The pharmaceutical compositions may be, for example, compositions for enteral, such as oral, rectal, aerosol inhalation or nasal administration, compositions for parenteral, such as intravenous or subcutaneous administration, or compositions for transdermal administration (e.g. passive or iontophoretic).

Preferably, the pharmaceutical compositions are adapted to oral or parenteral (especially intravenous, subcutaneous, intramuscular, or transdermal) administration. Intravenous and oral, first and foremost intravenous administration is considered to be of particular importance. Preferably the bisphosphonate active ingredient is in the form of a parenteral, most preferably an intravenous form.

The particular mode of administration and the dosage may be selected by the attending physician taking into account the particulars of the patient, especially age, weight, life style, activity level, hormonal status (e.g. post-menopausal) and bone mineral density as appropriate.

The dosage of the Agents of the Invention may depend on various factors, such as effectiveness and duration of action of the active ingredient, e.g. including the relative potency of the bisphosphonate used, mode of administration, warm-blooded species, and/or sex, age, weight and individual condition of the warm-blooded animal.

Normally the dosage is such that a single dose of the zoledronic acid, or a pharmaceutically acceptable salt thereof, or any hydrate thereof active ingredient from 0.005 - 20 mg/kg, especially 0.01 - 10 mg/kg, is administered to a warm-blooded animal weighing approximately 75kg.

"mg/kg" means mg drug per kg body weight of the mammal - including man - to be treated.

The dose mentioned above is typically administered intermittently with a period of at least 6 months between doses. The period between administrations may be longer, e.g. conveniently once per year, once per 18 months or once every 2 years, or even longer, or any period in between.

Formulations in single dose unit form contain preferably from about 1% to about 90%, and formulations not in single dose unit form contain preferably from about 0.1 % to about 20%, of the active ingredient. Single dose unit forms such as ampoules of infusion solution or solid for preparation of infusion solution doses, capsules, tablets or dragées contain e.g. from about 0.5 mg to about 500mg of the active ingredient. It will be appreciated that the actual unit dose used will depend upon the potency of the zoledronic acid, or a pharmaceutically acceptable salt thereof, or any hydrate thereof, the dosing interval and route of administration amongst other things. Thus the size of the unit dose is typically lower for more potent bisphosphonates and greater the longer the dosing interval. For example, for zoledronic acid a unit dose of from about 1 up to about 10 mg may be used for parenteral, e.g. intravenous, administration. A unit dose of from about 1 to about 5 mg may be used parenterally for dosing once every 6 months; whereas a dose of from about 2 up to about 10 mg may be used for once a year parenteral dosing.

Unit doses may be administered as a single or divided dose, i.e. a dose in which the unit dose is divided into two or more equal or unequal parts and in which the parts are administered to the patient at the same time, during overlapping time periods or at separate time points. When the unit dose is administered as a divided dose at separate time points, the interval between the separate administrations of the divided dose may be from hours, e.g. 1 hour, up to about 1 month (approximately 30 days). In accordance with the invention, the time interval between administration of the last part of the divided dose and administration of the first part of the next, following divided dose is at least 6 months or longer, e.g. about 1 year.

Thus, for example, a 10 mg unit dose may be administered as two equal 5 mg parts at an interval of about 1 week to about 1 month, e.g. about 2 weeks, between administration of the parts. Alternatively, for example, a 5 mg unit dose may be administered as two unequal parts of 4 mg and 1 mg (or 3 mg and 2 mg) at an interval of from 1 to 3 days to 1 to 3 weeks, e.g. about 1 week, between administration of the parts.

Pharmaceutical preparations for enteral and parenteral administration are, for example, those in dosage unit forms, such as dragées, tablets or capsules and also ampoules. They are prepared in a manner known *per se*, for example by means of conventional mixing, granulating, confectioning, dissolving or lyophilising processes. For example, pharmaceutical preparations for oral administration can be obtained by combining the active ingredient with solid carriers, where appropriate granulating a resulting mixture, and processing the mixture or granulate, if desired or necessary after the addition of suitable adjuncts, into tablets or dragée cores.

Suitable carriers are especially fillers, such as sugars, for example lactose, saccharose, mannitol or sorbitol, cellulose preparations, and also binders, such as starch pastes, using, for example, corn, wheat, rice or potato starch, gelatin, tragacanth, methylcellulose and/or polyvinylpyrrolidone and, if desired, disintegrators, such as the above-mentioned starches, also carboxymethyl starch, crosslinked polyvinylpyrrolidone, agar or alginic acid or a salt thereof, such as sodium alginate. Adjuncts are especially flow-regulating agents and lubricants, for example silicic acid, talc, stearic acid or salts thereof, such as magnesium or calcium stearate, and/or polyethylene glycol. Dragee cores are provided with suitable coatings that may be resistant to gastric juices, there being used, inter alia, concentrated sugar solutions that optionally contain gum arabic, talc, polyvinylpyrrolidone, polyethylene glycol and/or titanium dioxide, or lacquer solutions in suitable organic solvents or solvent mixtures or, to produce coatings that are resistant to gastric juices, solutions of suitable cellulose preparations, such as acetylcellulose phthalate or hydroxypropylmethylcellulose phthalate. Colouring substances or pigments may be added to the tablets or dragee coatings, for example for the purpose of identification or to indicate different doses of active ingredient.

Other orally administrable pharmaceutical preparations are dry-filled capsules made of gelatin, and also soft, sealed capsules made of gelatin and a plasticiser, such as glycerol or sorbitol. The dry-filled capsules may contain the active ingredient in the form of a granulate, for example in admixture with fillers, such as lactose, binders, such as starches, and/or glidants, such as talc or magnesium stearate, and, where appropriate, stabilisers. In soft capsules the active ingredient is preferably dissolved or suspended in suitable liquids, such as fatty oils, paraffin oil or liquid polyethylene glycols, it being possible also for stabilisers to be added.

Parenteral formulations are especially injectable fluids that are effective in various manners, such as, intramuscularly, intraperitoneally, intranasally, intradermally, subcutaneously or preferably intravenously. Such fluids are preferably isotonic aqueous solutions or suspensions which can be prepared before use, for example from lyophilised preparations which contain the active ingredient alone or together with a pharmaceutically acceptable carrier, or from solution concentrates. The pharmaceutical preparations may be sterilised and/or contain adjuncts, for example preservatives, stabilisers, wetting agents and/or emulsifiers, solubilisers, salts for regulating the osmotic pressure and/or buffers.

Suitable formulations for transdermal application include an effective amount of the active ingredient with carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the active ingredient to the skin of the host at a controlled and predetermined rate over a prolonged period of time, and means to secure the device to the skin.

The following Examples illustrate the invention described hereinbefore.

In the following Examples the term "active ingredient" is to be understood as being zoledronic acid, or a pharmaceutically acceptable salt thereof, or any hydrate thereof derivative mentioned above as being useful according to the present invention.

### Example 2: Monolith adhesive transdermal system, containing as active ingredient, for example, 1-hydroxy-2-(imidazol-1-yl)-ethane-1,1-diphosphonic acid:

### Composition:

| | |
|---|---|
| polyisobutylene (PIB) 300 | 5.0 g |
| (Oppanol B1, BASF) | |
| PIB 35000 | 3.0 g |
| (Oppanol B10, BASF) | |
| PIB 1200000 | 9.0 g |
| (Oppanol B 100, BASF) | |
| hydrogenated hydrocarbon resin | 43.0 g |
| (Escorez 5320, Exxon) | |
| 1-dodecylazacycloheptan-2-one | 20.0 g |
| (Azone, Nelson Res., Irvine/CA) | |
| active ingredient | 20.0 g |
| Total | 100.0 g |

### Preparation:

The above components are together dissolved in 150 g of special boiling point petroleum fraction 100-125 by rolling on a roller gear bed. The solution is applied to a polyester film (Hostaphan, Kalle) by means of a spreading device using a 300mm doctor blade, giving a coating of about 75 g/m². After drying (15 minutes at 60°C), a silicone-treated polyester film (thickness 75 mm, Laufenberg) is applied as the peel-off film. The finished systems are punched out in sizes in the wanted form of from 5 to 30cm² using a punching tool. The complete systems are sealed individually in sachets of aluminised paper.

Example 3: Vial containing 1.0 mg dry, lyophilized 1-hydroxy-2-(imidazol-1-yl)ethane-1,1-diphosphonic acid (mixed sodium salts thereof). After dilution with 1 ml of water, a solution (concentration 1 mg/ml) for i.v. infusion is obtained.

### Composition:

| | |
|---|---|
| active ingredient (free diphosphonic acid) | 1.0 mg |
| mannitol | 46.0 mg |
| Trisodium citrate x 2 H₂O | ca. 3.0 mg |
| water | 1 ml |
| water for injection | 1 ml . |

In 1 ml of water, the active ingredient is titrated with trisodium citrate x 2 H₂O to pH 6.0. Then, the mannitol is added and the solution is lyophilized and the lyophilisate filled into a vial.

Example 4: Ampoule containing active ingredient, for instance disodium pamidronate pentahydrate dissolved in water. The solution (concentration 3 mg/ml) is for i.v. infusion after dilution.

### Composition:

| | |
|---|---|
| active ingredient ( ≙ 5.0 mg of anhydrous active ingredient) | 19.73 mg |
| mannitol | 250 mg |
| water for injection | 5 ml . |

### Example 5 Treatment of Patients

"A Multinational, Randomized, Double-Blind, Placebo-Controlled, Parallel-Group, Dose-Ranging, Safety and Efficacy Trial With Intravenous Bolus Injections of Zoledronate In the Treatment of Postmenopausal Osteoporosis"

This was a dose and dose regimen-finding 12 month trial of iv zoledronic acid in patients with postmenopausal osteoporosis. Three hundred and fifty one patients were randomized to six study arms. Patients who had recent exposure to bone active drugs, e.g. bisphosphonates, estrogen, calcitonin, raloxifene, or a history of metabolic bone diseases were excluded. All patients were evaluated at baseline and in 3-monthly visits. Zoledronic acid or placebo was administered as a bolus iv injection into a peripheral vein over 5 minutes at every visit.

Efficacy was ascertained by measurement of percent change from baseline in bone mineral density (BMD) measured by dual energy X-ray absorptiometry (DEXA) as compared to placebo, at 6, 9, and 12 months.

As a special safety measure trans-iliac bone biopsies were obtained in a subset of patients from all study arms at 12 months, and X-rays of the thoracic and lumbar spine from all study participants were evaluated at baseline and at 12 months for the occurrence of incident vertebral fractures.

Additionally, the degree and duration of suppression of biochemical markers of bone turnover - parathyroid hormone (PTH), bone specific alkaline phosphatase (BSAP), serum C-telopeptide (CTX), serum osteocalcin, urine N-telopeptide (NTX)/creatinine ratio, urine deoxypyridinoline (d-pyd)/creatinine ratio, urine pyridinoline (pyd)/creatinine ratio - was obtained every 3 months and measured in a central laboratory.

### Study Arms

### · Placebo

· 0.25 mg zoledronic acid every 3 months
· 0.5 mg zoledronic acid every 3 months
· 1.0 mg zoledronic acid every 3 months
· 2.0 mg zoledronic acid every 6 months
· 4.0 mg zoledronic acid every 12 months

The 12 month results showed that all treatment arms demonstrated a percent change from baseline in BMD significantly (p < 0.001) greater than placebo and not dissimilar one from another.

| Summary of stepwise multiple comparisons of the active doses of zoledronate versus placebo for percent change from baseline in bone mineral density of the lumbar spine; postero anterior (L1-L4) at 12 months Confirmatory analysis ITT population | | | | | |
|---|---|---|---|---|---|
| Step Number | Most significant contrast | Difference | Standard error of difference | Lower 97.5% confidence limit | p-value |
| 1 | zoledronate 4 x 0.25 mg - placebo | 5.1 | 0.55 | 3.7 | <0.001 |
| 2 | zoledronate 4 x 0.5 mg - placebo | 4.9 | 0.56 | 3.5 | <0.001 |
| 3 | zoledronate 1 x 4.0 mg - placebo | 4.6 | 0.53 | 3.3 | <0.001 |
| 4 | zoledronate 4 x 1.0 mg - placebo | 4.5 | 0.55 | 3.2 | <0.001 |
| 5 | zoledronate 2 x 2.0 mg - placebo | 4.2 | 0.57 | 3.1 | <0.001 |

| | | | | | |
|---|---|---|---|---|---|
| Note: Stepwise multiple comparison of the active doses of zoledronate versus placebo at a one-sided multiple alpha level of 2.5% adjusting for multiple comparisons according to Marcus, Peritz and Gabriel (1976) | | | | | |

Bone mineral density increased compared to placebo at the spine, hip, distal radius, and "total body". Suppression of biochemical markers of bone formation and bone resorption confirmed and supported the BMD results, demonstrating suppression of bone turnover to the pre-menopausal level throughout the 6 and 12 month dosing intervals.

The BMD data indicate that zoledronic acid dose administration as infrequent as every 6 or 12 months can safely result in a statistically significant and medically relevant bone mass increase. It is believed that these data further indicate that a continued preservation of new bone beyond one year, without additional dose administration, is likely or that further bone mass increase is possible. It is also believed that re-treatment in additional cycles of every 6 month, 12 month, or less frequent dose administration will lead to further BMD increase. A reduction of risk of osteoporotic fracture is expected to accompany the bone mass increases.

## Claims

1. Zoledronic acid or a pharmaceutically acceptable salt thereof or any hydrate thereof for use in a method of treating osteoporosis in which the zoledronic acid or the pharmaceutically acceptable salt therefore or the hydrate thereof is administered intravenously and intermittently and in which the period between administrations is at least about 6 months.

2. Zoledronic acid or a pharmaceutically acceptable salt thereof or any hydrate thereof for use according to claim 1 wherein the osteoporosis is postmenopausal osteoporosis.

3. Zoledronic acid or a pharmaceutically acceptable salt thereof or any hydrate thereof for use according to any one of the preceding claims wherein the use in the treatment of osteoporosis is for reducing the risk of osteoporotic fractures.

4. Zoledronic acid or a pharmaceutically acceptable salt thereof or any hydrate thereof for use according to claim 1 wherein the osteoporosis is male osteoporosis.

5. Zoledronic acid or a pharmaceutically acceptable salt thereof or any hydrate thereof for use according to claim 1 wherein the osteoporosis is corticosteroid-induced osteoporosis.

6. Zoledronic acid or a pharmaceutically acceptable salt thereof or any hydrate thereof for use according to claim 1 in which the period between administrations is at least about a year.

7. Zoledronic acid or a pharmaceutically acceptable salt thereof or any hydrate thereof for use according to any one of the preceding claims wherein the period between administrations is one year.

8. A composition comprising zoledronic acid, a pharmaceutically acceptable salt thereof or any hydrate thereof for use in a method of treating osteoporosis, wherein the composition is administered intermittently and in which the period between administrations is at least about 6 months.

9. A composition for use according to claim 8 wherein the osteoporosis is postmenopausal osteoporosis.

10. A composition for use according to claim 8 or claim 9 wherein the use in the treatment of osteoporosis is for reducing the risk of osteoporotic fractures.

11. A composition for use according to claim 8 wherein the osteoporosis is male osteoporosis.

12. A composition for use according to claim 8 wherein the osteoporosis is corticosteroid-induced osteoporosis.

13. A composition for use according to claim 8 in which the period between administrations is at least about a year.

14. A composition for use according to any one of claims 8-13 for intermittent administration in which the period between administrations is one year.

15. A composition for use according to any one of claims 8-14 for intermittent parenteral administration.

16. A composition for use according to any one of claims 8-15 for intermittent intravenous administration.

17. Zoledronic acid, a pharmaceutically acceptable salt thereof or any hydrate thereof in a unit dose of from about 2 up to about 10 mg for use in a method of treating conditions of abnormally increased bone turnover, in which the zoledronic acid or the pharmaceutically acceptable salt therefore or the hydrate thereof is administered intravenously and intermittently in which the period between administrations is about a year.

## Patentansprüche

1. Zoledronsäure, oder pharmazeutisch unbedenkliches Salz davon oder beliebiges Hydrat davon für die Verwendung in einem Verfahren zur Behandlung von Osteoporose, bei dem die Zoledronsäure oder das pharmazeutisch unbedenkliche Salz davon oder das Hydrat davon intravenös und intermittierend verabreicht wird und bei dem der Zeitraum zwischen den Verabreichungen mindestens ungefähr 6 Monate beträgt.

2. Zoledronsäure oder pharmazeutisch unbedenkliches Salz davon oder beliebiges Hydrat davon für die Verwendung nach Anspruch 1, wobei es sich bei der Osteoporose um Postmenopause-Osteoporose handelt.

3. Zoledronsäure oder pharmazeutisch unbedenkliches Salz davon oder beliebiges Hydrat davon für die Verwendung nach einem der vorhergehenden Ansprüche, wobei die Verwendung in der Behandlung von Osteoporose der Verringerung des Risikos von Osteoporose-Frakturen dient.

4. Zoledronsäure oder pharmazeutisch unbedenkliches Salz davon oder beliebiges Hydrat davon für die Verwendung nach Anspruch 1, wobei es sich bei der Osteoporose um Osteoporose des Mannes handelt.

5. Zoledronsäure oder pharmazeutisch unbedenkliches Salz davon oder beliebiges Hydrat davon für die Verwendung nach Anspruch 1, wobei es sich bei der Osteoporose um Corticosteroid-induzierte Osteoporose handelt.

6. Zoledronsäure oder pharmazeutisch unbedenkliches Salz davon oder beliebiges Hydrat davon für die Verwendung nach Anspruch 1, wobei der Zeitraum zwischen den Verabreichungen mindestens ungefähr ein Jahr beträgt.

7. Zoledronsäure oder pharmazeutisch unbedenkliches Salz davon oder beliebiges Hydrat davon für die Verwendung nach einem der vorhergehenden Ansprüche, wobei der Zeitraum zwischen den Verabreichungen ein Jahr beträgt.

8. Zusammensetzung, umfassend Zoledronsäure, ein pharmazeutisch unbedenkliches Salz davon oder ein beliebiges Hydrat davon für die Verwendung in einem Verfahren zur Behandlung von Osteoporose, wobei die Zusammensetzung intermittierend verabreicht wird und wobei der Zeitraum zwischen den Verabreichungen mindestens ungefähr 6 Monate beträgt.

9. Zusammensetzung für die Verwendung nach Anspruch 8, wobei es sich bei der Osteoporose um Postmenopause-Osteoporose handelt.

10. Zusammensetzung für die Verwendung nach Anspruch 8 oder Anspruch 9, wobei die Verwendung in der Behandlung von Osteoporose der Verringerung des Risikos von Osteoporose-Frakturen dient.

11. Zusammensetzung für die Verwendung nach Anspruch 8, wobei es sich bei der Osteoporose um Osteoporose des Mannes handelt.

12. Zusammensetzung für die Verwendung nach Anspruch 8, wobei es sich bei der Osteoporose um Corticosteroid-induzierte Osteoporose handelt.

13. Zusammensetzung für die Verwendung nach Anspruch 8, wobei der Zeitraum zwischen den Verabreichungen mindestens ungefähr ein Jahr beträgt.

14. Zusammensetzung für die Verwendung nach einem der Ansprüche 8-13 für die intermittierende Verabreichung, wobei der Zeitraum zwischen den Verabreichungen ein Jahr beträgt.

15. Zusammensetzung für die Verwendung nach einem der Ansprüche 8-14 für die intermittierende parenterale Verabreichung.

16. Zusammensetzung für die Verwendung nach einem der Ansprüche 8-15 für die intermittierende intravenöse Verabreichung.

17. Zoledronsäure, pharmazeutisch unbedenkliches Salz davon oder ein beliebiges Hydrat davon in Einzeldosisform von ungefähr 2 bis ungefähr 10 mg für die Verwendung in einem Verfahren zur Behandlung von Erkrankungen, die einen abnorm erhöhten Knochenumsatz umfassen, wobei die Zoledronsäure oder das pharmazeutisch unbedenkliche Salz davon oder das Hydrat davon intravenös und intermittierend verabreicht wird, wobei der Zeitraum zwischen den Verabreichungen ungefähr ein Jahr beträgt.

## Revendications

1. Acide zolédronique ou un sel pharmaceutiquement acceptable de celui-ci ou un hydrate quelconque de celui-ci pour utilisation dans un procédé de traitement de l'ostéoporose dans lequel de l'acide zolédronique ou le sel pharmaceutiquement acceptable de celui-ci ou l'hydrate de celui-ci est administré par voie intraveineuse et de façon intermittente et dans lequel la période entre les administrations est d'au moins environ 6 mois.

2. Acide zolédronique ou un sel pharmaceutiquement acceptable de celui-ci ou un hydrate quelconque de celui-ci pour utilisation selon la revendication 1, l'ostéoporose étant l'ostéoporose post-ménopause.

3. Acide zolédronique ou un sel pharmaceutiquement acceptable de celui-ci ou un hydrate quelconque de celui-ci pour utilisation selon l'une quelconque des revendications précédentes, l'utilisation dans le traitement de l'ostéoporose étant pour réduire le risque de fractures ostéoporotiques.

4. Acide zolédronique ou un sel pharmaceutiquement acceptable de celui-ci ou un hydrate quelconque de celui-ci pour utilisation selon la revendication 1, l'ostéoporose étant l'ostéoporose masculine.

5. Acide zolédronique ou un sel pharmaceutiquement acceptable de celui-ci ou un hydrate quelconque de celui-ci pour utilisation selon la revendication 1, l'ostéoporose étant l'ostéoporose induite par les corticostéroïdes.

6. Acide zolédronique ou un sel pharmaceutiquement acceptable de celui-ci ou un hydrate quelconque de celui-ci pour utilisation selon la revendication 1, la période entre administrations étant d'au moins environ un an.

7. Acide zolédronique ou un sel pharmaceutiquement acceptable de celui-ci ou un hydrate quelconque de celui-ci pour utilisation selon l'une quelconque des revendications précédentes, la période entre administrations étant d'un an.

8. Composition comprenant de l'acide zolédronique ou un sel pharmaceutiquement acceptable de celui-ci ou un hydrate quelconque de celui-ci pour utilisation dans un procédé de traitement de l'ostéoporose, la composition étant administrée de façon intermittente et la période entre administrations étant d'au moins environ 6 mois.

9. Composition pour utilisation selon la revendication 8, l'ostéoporose étant l'ostéoporose post-ménopause.

10. Composition pour utilisation selon la revendication 8 ou la revendication 9, l'utilisation dans le traitement de l'ostéoporose étant destinée à réduire le risque de fractures ostéoporotiques.

11. Composition pour utilisation selon la revendication 8, l'ostéoporose étant l'ostéoporose masculine.

12. Composition pour utilisation selon la revendication 8, l'ostéoporose étant l'ostéoporose induite par les corticostéroïdes.

13. Composition pour utilisation selon la revendication 8, la période entre administrations étant d'au moins environ un an.

14. Composition pour utilisation selon l'une quelconque des revendications 8 à 13 pour administration intermittente, la période entre administrations étant d'un an.

15. Composition pour utilisation selon l'une quelconque des revendications 8 à 14 pour administration parentérale intermittente.

16. Composition pour utilisation selon l'une quelconque des revendications 8 à 15 pour administration intraveineuse intermittente.

17. Acide zolédronique, sel pharmaceutiquement acceptable de celui-ci ou hydrate quelconque de celui-ci dans une dose unitaire d'environ 2 jusqu'à 10 mg pour utilisation dans un procédé de traitement d'affections de turnover osseux anormalement augmenté, dans lequel l'acide zolédronique ou le sel pharmaceutiquement acceptable de celui-ci ou l'hydrate de celui-ci étant administré par voie intraveineuse et de façon intermittente, la période entre administrations étant d'environ un an.
